Europäisches Patentamt

European Patent Office

Office européen des brevets

(11) Publication number: **0 420 193 A2**

# EUROPEAN PATENT APPLICATION

(21) Application number: 90118475.4

(22) Date of filing: 26.09.90

(51) Int. Cl.5: **A61K 31/17**, A61K 33/24,
A61K 31/70, A61K 31/505,
A61K 31/675, A61K 31/195,
//(A61K33/24,31:17),
(A61K31/70,31:17),
(A61K31/505,31:17),
(A61K31/675,31:17),
(A61K31/195,31:17)

The title of the invention has been amended
(Guidelines for Examination in the EPO, A-III,
7.3).

(30) Priority: 27.09.89 US 413506

(43) Date of publication of application:
03.04.91 Bulletin 91/14

(84) Designated Contracting States:
AT BE CH DE DK ES FR GB GR IT LI LU NL SE

(71) Applicant: **E.R. SQUIBB & SONS, INC.**
**P.O.Box 4000**
**Princeton New Jersey 08543-4000(US)**

(72) Inventor: **Mascoli, Carmine C.**
**608 Station Ave.**
**Langhorne, PA(US)**
Inventor: **DeGroff, Robert C.**
**145 Water Crest Drive**
**Doylestown, PA(US)**
Inventor: **Henry, Edward V.**
**1 Prospect St.**
**Peapack, NJ(US)**
Inventor: **Balwani, Gul P.**
**57 Bradford Lane**
**Plainsboro, NJ(US)**

(74) Representative: **Vossius & Partner**
**Siebertstrasse 4 P.O. Box 86 07 67**
**W-8000 München 86(DE)**

(54) Use of hydroxyurea or 1-ethyl-hydroxyurea for the suppression of bone marrow cells before transplantation.

(57) A novel method for the suppression of bone marrow cells, particularly suitable for the substantial suppression of bone marrow cells preparatory to bone marrow transplantation in leukemia patients, is disclosed. This method entails the administration of hydroxyurea of 1-ethyl-1-hydroxyurea sufficient to provide the desired level of cell suppression.

EP 0 420 193 A2

# USE OF HYDROXYUREA OR 1-ETHYL-1-HYDROXYUREA FOR THE SUPPRESSION OF BONE MARROW

Successful treatment modalities for leukemia typically include systemic chemotherapy which may be complemented with radiation therapy, corticosteroids and/or antibiotics. Antineoplastic agents suitable for such chemotherapy include cyclophosphamide, cytarabine, melphalan, cisplatin, carmustine, etoposide, hydroxyurea, vincristine and the like. In some cases, bone marrow transplantation is advocated. For example, a bone marrow transplant may be appropriate for patients in periods of remission from chronic myelocytic leukemia, chronic lymphocytic leukemia, acute myeloblastic leukemia, acute myelogenous leukemia and acute lymphoblastic leukemia.

Conditioning of patients prior to bone marrow transplant involves the substantial elimination of all cellular elements of the recipient's marrow prior to transplant. Some of the antineoplastic agents described above have been used to affect such immunosuppression such as cyclophosphamide, melphalan and atoposide.

Indeed, bone marrow suppression is considered an adverse reaction of most antineoplastic agents, but attempts to intentionally employ this characteristic to suppress bone marrow in a safe, controlled procedure have been fraught with difficulties. Determining proper dosages is extremely difficult in that a relatively high degree of precision is required. Too high a dose of the bone marrow suppressing agent unduly subjects the patient to drug side effects whereas too low of a dose can adversely affect the transplant operation. Further, even at low doses, labeled warnings for agents such as methotrexate, carmustine and cyclophosphamide include cardiac toxicity, secondary malignancies, pulmonary toxicity, gastrointestinal toxicity, nephrotoxicity and hepatotoxicity.

In accordance with the present invention hydroxyurea or 1-ethyl-1-hydroxyurea in an amount sufficient to substantially suppress the bone marrow is used. This can be accomplished by administering a fixed dose schedule of bolus injections or by intravenous infusion of hydroxyurea or 1-ethyl-1-hydroxyurea.

The present invention provides the use of hydroxyurea or 1-ethyl-1-hydroxyurea for suppressing bone marrow. By intravenously infusing and titrating hydroxyurea or 1-ethyl-1-hydroxyurea, bone marrow supression to any desired level, including, substantial elimination of the bone marrow, can be achieved in patients requiring such treatment. U. S. Patent 3,968,249 discloses the use of hydroxyurea or 1-ethyl-1-hydroxyurea to treat malignant noeplastic diseases. This '249 patent effectively covers the presently approved uses for hydroxyurea and methods disclosed therein are for treating and ameliorating the symptoms of malignant noeplastic diseases. The primary warning with these prior uses of hydroxyurea is that this drug (like many other antinoeplastics) causes bone marrow supression. Unexpectedly continuous IV infusion of hydroxyurea or 1-ethyl-1-hydroxyurea provides that this previously avoided can be harnessed for a positive purpose.

While the prior art antinoeplastics have been tried in the field of bone marrow transplants, the non-marrow related toxicities of these agents has seriously limited their use. The relatively modest profile of non-marrow related toxicities for hydroxyurea and 1-ethyl-1-hydroxyurea as compared to prior art antineoplastic agents affords a procedure with less adverse side effects and the ability to use higher doses of medication. Therefore, enhanced bone marrow suppression with less side effects, whether by fixed dose or intravenous titration, is provided by the present invention.

In practicing the present method of bone marrow suppression preparatory to bone marrow transplant, the hydroxyurea should be given by continuous IV infusion using a varied dosage range, titrating the medication until the maximum tolerated dosage is achieved, or until the modified World Health Organizations toxicity criteria are observed. The dosage should be titrated until myeloid suppression or bone marrow amelioration is achieved.

To prepare the pharmaceutical compositions to be administered according to this invention the hydroxyurea or 1-ethyl-1-hydroxyurea is incorporated in various pharmaceutical preparations by mixing effective amounts thereof with a suitable proportion of a compatible non-toxic pharmaceutical carrier. Illustratively, the formulation may comprise isotonic liquid preparations for parenteral administration, prepared by suspending the compound in water and adjusting the salinity of the resulting solution. Any other dosage forms suitable for parenteral administration can also be employed. Thus, compositions may be prepared using an aqueous-alcohol vehicle.

The proportions of active ingredient in the compositions administered according to the present invention may be varied over a substantial range, subject to the practical limitation that sufficient proportion of active ingredient is present to provide a suitable dosage.

The continuous IV hydroxyurea or 1-ethyl-1-hydroxyurea administration, or fixed dose bolus injection administration, of the present method may also be complemented with other modalities to enhance/hasten the desired bone marrow suppression. For example, the present method can be carried out in conjunction with radiation therapy or with the concomitant administration (orally or parenterally) of other antineoplastic agents, e.g., cyclophosphamide, cytarabine, melphalon, cisplatin, carmustine and etoposide. Concomitant or intermittent irradiation therapy or hormone therapy may also be incorporated into the present method.

The present procedure is suitable in any treatment which would require the suppression of bone marrow cells and is suitable as a preparation for bone marrow transplants. This procedure is especially suitable for the substantial elimination of bone marrow cells preparatory to bone marrow transplantation in patients suffering from leukemia, particularly chronic myelocytic leukemia, chronic lymphocytic leukemia, acute myeloblastic leukemia, acute myelogenous leukemia and acute lymphoblastic leukemia.

Preparations for use in the present method are further illustrated by the following examples, however, this invention should in no way be limited by the details described therein.

Example 1

An aqueous sterile suspension of hydroxyurea providing an effective dose of 50 mg of hydroxyurea per ml was prepared by combining the following ingredients:

| Hydroxyurea | 50.5 g |
|---|---|
| Benzyl alcohol | 9.0 g |
| Sodium chloride | 6.6 g |
| Carboxymethylcellulose | 5.5 g |
| Methylcellullose | 0.75 g |
| Water q.s. | 1.0 liter |

The sodium chloride, carboxymethylcellulose and methylcellulose was added to 100 ml of water with attendant stirring. The hydroxyurea and benzyl alcohol was then added with agitation. Sufficient water was then added to bring the volume to one liter. The resultant suspension was then metered into vials of the selected size as, for example, 10 ml vials, from which it can be withdrawn for therapeutic application as by intramuscular administration.

Example 2

Hydroxyurea (20.0 g) was added to a solution of 528 g of citric acid (anhydrous) in 160 ml of water and the mixture was stirred until complete solution was obtained. The solution was aseptically filtered, frozen at -30°C and lyophilized at 50-100 microns vacuum. The resulting solid mixture was filled into sealed vials each containing 2.0 g of hydroxyurea.

## Claims

1. Use of hydroxyurea and/or 1-ethyl-1-hydroxyurea for the preparation of a pharmaceutical composition for the suppression or substantial elimination of bone marrow cells.

2. Use according to claim 1 wherein the composition is suitable for substantial elimination of bone marrow cells preparatory to bone marrow transplantation.

3. Use according to any of claims 1 or 2 wherein the composition is suited for bolus injection.

4. Use according to any of claims 1 or 2 wherein the composition is administered in a fixed dose schedule of bolus injections.

5. Use according to any of claims 1 or 2 wherein the composition is administered in the form of an intravenous infusion.

6. Use according to any of claims 1 or 2 wherein the composition is suitable for treatment of leukemia.

7. Use according to claim 6 wherein said leukemia is chronic myelocytic leukemia, chronic lymphocytic leukemia, acute myeloblastic leukemia, acute myelogenous leukemia or acute lymphoblastic leukemia.

8. Use according to any of claims 1 or 2 wherein the composition is suited for the concomitant use of one or more antineoplastic agents.

9. Use according to claim 8 wherein said antineoplastic agents are cyclophosphamide, cytarabine, melphalon, cisplatin, carmustine or etoposide.